(19) Europäisches Patentamt
European Patent Office
Office européen des brevets

(11) Veröffentlichungsnummer: **0 022 241**
**B1**

(12) # EUROPÄISCHE PATENTSCHRIFT

(45) Veröffentlichungstag der Patentschrift: **10.11.82**

(51) Int. Cl.³: **C 07 C 76/06,** C 07 C 79/12

(21) Anmeldenummer: **80103727.6**

(22) Anmeldetag: **01.07.80**

(54) **Verfahren zur Entfernung von Nitrosierungsmittel(n) aus nitrierten aromatischen Verbindungen.**

(30) Priorität: **04.07.79 DE 2926947**

(43) Veröffentlichungstag der Anmeldung: **14.01.81 Patentblatt 81/2**

(45) Bekanntmachung des Hinweises auf die Patenterteilung: **10.11.82 Patentblatt 82/45**

(84) Benannte Vertragsstaaten: **CH DE FR GB IT LI NL**

(56) Entgegenhaltungen:

**DE-A-2 131 561**
**DE-A-2 746 787**
**DE-A-2 831 119**
**DE-A-2 835 530**
**DE-A-2 837 529**
**DE-A-2 840 551**
**DE-B-1 092 459**
**DE-B-2 635 695**
**US-A-2 257 093**
**US-A-4 096 195**

(73) Patentinhaber: **HOECHST AKTIENGESELLSCHAFT, Postfach 80 03 20, D-6230 Frankfurt/Main 80 (DE)**

(72) Erfinder: **Habig, Kurt, Dr., Hundertmorgenring 98, D-6082 Mörfelden (DE)**
Erfinder: **Baessler, Konrad, Dr., Drosselweg 1, D-6230 Frankfurt am Main 80 (DE)**

**EP 0 022 241 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Entfernung von Nitrosierungsmittel(n) aus nitrierten aromatischen Verbindungen

Unter «nitrierten aromatischen Verbindungen» werden im folgenden aromatische Nitroverbindungen verstanden, welche durch Nitrierung entsprechender aromatischer Ausgangsverbindungen mit konzentrierter Salpetersäure oder mit Nitriersäure (konzentrierte Salpetersäure/Schwefelsäure) und übliche Aufarbeitung hergestellt worden sind. Aromatische Nitroverbindungen, welche noch durch auf die Nitrierung folgende Reaktionsschritte weiter umgesetzt wurden, sollen unter «nitrierten aromatischen Verbindungen» hier nicht verstanden werden.

Nitrierte aromatische Verbindungen sind End- und Zwischenprodukte auf verschiedenen Sachgebieten, insbesondere auf dem Sprengstoff-, Pharma- und Pflanzenschutzsektor.

Eine als Zwischenprodukt auf dem Pflanzenschutzsektor wichtige nitrierte aromatische Verbindung ist z.B. das durch Nitrierung von p-Chlor-benzotrifluorid erhältliche 4-Chlor-3,5--dinitrobenzotrifluorid, welches u.a. durch Umsetzung mit Di-n-propylamin das Herbizid Trifluralin (= 4-Di-n-propylamino-3,5-dinitrobenzotrifluorid) liefert. Diese Synthese lässt sich durch folgendes Reaktionsschema wiedergeben:

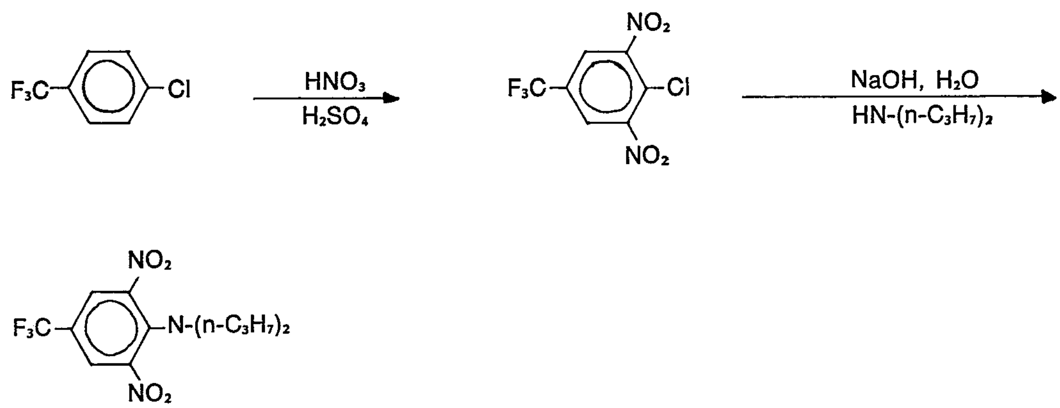


Mit Hilfe moderner Analysenmethoden hat man in dem so hergestellten Trifluralin nun jedoch geringe Mengen von Nitrosaminen gefunden, die zumindest bei Tieren karzinogen wirken können und deswegen ausserordentlich unerwünscht sind (siehe z.B. DE-OS 2 835 530). Die Bildung dieser Nitrosamine dürfte auf der Nitrosierung des in der letzten Reaktionsstufe eingesetzten Di-n-propylamins durch Nitrosierungsmittel beruhen, die in der Vorstufe des Trifluralins — im 4-Chlor-3,5-dinitrobenzotrifluorid — von der Nitrierungsreaktion her in geringer Menge vorhanden zu sein scheinen.

Man hat daher bereits verschiedene Verfahren entwickelt, um die Nitrosamine aus dem Trifluralin wieder so vollständig wie möglich zu entfernen. Nach dem Verfahren z.B. der DE-OS 2 831 119 geschieht dies durch Behandlung mit 20-38%iger Salzsäure oder mit gasförmigem Chlorwasserstoff in flüssiger Phase bei Temperaturen bis zu 140°C.

Eine Behandlung mit Brom, Chlor, N-Bromsuccinimid, N-Chlorsuccinimid, Bromchlorid, Pyridinperbromid und/oder Pyridiniumbromidperbromid zum Zwecke ebenfalls der Verminderung der Nitrosaminkonzentration wird in der DE-OS 2 835 530 und eine Behandlung mit Phosphor- oder Schwefelhalogeniden und -oxihalogeniden oder mit $TiCl_4$ in der DE-OS 2 837 529 beschrieben.

Diese Methoden führen zunächst zu einem befriedigenden Abfall der Nitrosaminwerte, doch sollen diese Werte bei längerer Reaktionszeit wieder ansteigen; wenn die Reaktionszeit daher nicht genau optimiert wird, kann durchaus der Fall eintreten, dass die nach diesen Verfahrensweisen an sich möglichen niedrigen Nitrosaminwerte nicht erhalten werden. Ausserdem bedeuten diese Verfahrensweisen einen zusätzlichen Arbeitsgang mit der Notwendigkeit der Aufarbeitung und gegebenenfalls Rückgewinnung der eingesetzten Reagentien.

Man hat auch schon versucht, es gar nicht erst zur Bildung der Nitrosamine kommen zu lassen, indem man aus der nitrierten Vorstufe des Trifluralins — dem 4-Chlor-3,5-dinitrobenzotrifluorid — das darin als Nebenprodukt enthaltene Nitrosierungsmittel soweit wie möglich entfernt (DE-OS 2 840 551). Dies geschieht durch eine Behandlung des genannten Vorproduktes

a) mit einer wässrigen Lösung einer anorganischen Base von einer Temperatur von 50 bis 100°C und — gegebenenfalls nach Abtrennung des so behandelten Produkts —

b) Hindurchleiten eines Inertgases (z.B. Luft) durch eine wässrige Mischung des Produkts bei der gleichen Temperatur und

c) gegebenenfalls Wiederholung der Stufen a) und/oder b) beliebige Male.

Durch diese Verfahrensweise wird der Gehalt an Nitrosierungsmittel(n) in dem 4-Chlor-3,5-dinitrobenzotrifluorid offensichtlich derart erniedrigt, dass bei der nachfolgenden Umsetzung mit Di-n-propylamin in dem dann erhaltenen Trifluralin ähnlich niedrige Nitrosaminwerte erhalten werden wie nach den bekannten Verfahrensweisen der direkten Nitrosaminentfernung aus dem Trifluralin. Das Verfahren zur Entfernung von Nitrosierungsmittel(n) aus der Trifluoralin-Vorstufe gemäss DE-OS 2 840 551 erscheint zwar — weil im Trifluralin dann kaum etwa ein Wiederanstieg des Nitrosaminwertes zu befürchten ist — günstiger als die Verfahrensweisen der DE-OSen 2 831 119, 2 835 530 und 2 837 529, doch ist insbesondere die Notwendigkeit von gegebenenfalls mehreren Arbeitsgängen auch hier nachteilig.

Zur Vermeidung dieses Nachteils wurde vorgeschlagen (Patentanmeldung P 29 20 448.6), das 4-Chlor-3,5-dinitrobenzotrifluorid einer Wasserbehandlung bei erhöhter Temperatur zu unterziehen, bei der man das für die Wasserbehandlung verwendete Wasser zumindest teilweise wieder dampfförmig entfernt. Die Wasserbehandlung wird zweckmässig in der Weise durchgeführt, dass man Wasserdampf durch das flüssige 4-Chlor-3,5-dinitrobenzotrifluorid leitet oder dass man diese Verbindung mit Wasser erhitzt und das Wasser gegebenenfalls unter schwachem Vakuum bei Temperaturen zwischen etwa 95 und 100°C abdestilliert. Durch diese Wasserbehandlung wird das 4-Chlor-3,5-dinitrobenzotrifluorid von Nitrosierungsmittel(n) sehr weitgehend bis praktisch vollständig befreit — eine Verfahrensweise, die sich über diesen Spezialfall hinaus ziemlich allgemein auch auf die Entfernung von Nitrosierungsmittel(n) aus nitrierten aromatischen Verbindungen anwenden lässt.

In dem Bestreben, die Verfahren des Standes der Technik zur Herstellung eines möglichst nitrosaminfreien Trifluralins weiter zu verbessern — sei es durch Entfernung der Nitrosamine in dem fertigen Trifluralin oder durch Vermeidung der Bildung dieser Nitrosamine — und diese Verbesserung auch möglichst allgemein anwenden zu können, wurde nun gefunden, dass dieses Ziel in ausserordentlich einfacher und befriedigender Weise dadurch erreicht werden kann, dass man das 4-Chlor-3,5-dinitrobenzotrifluorid — oder allgemein: die nitrierte aromatische Verbindung — aus dem diese Verbindung in geschmolzenem Zustand enthaltenden Nitrieransatz durch Abkühlen vorzugsweise unter Rühren auskristallisieren lässt. Das auskristallisierende 4-Chlor-3,5-dinitrobenzotrifluorid bzw. die auskristallisierende nitrierte aromatische Verbindung enthält praktisch keine Nitrosierungsmittel mehr.

Es war sehr überraschend, dass nitrierte aromatische Verbindungen ohne Abtrennung der organischen von der wässrigen Schicht durch Kristallisation direkt aus dem Nitrieransatz in praktisch nitrosierungsmittelfreier Form erhalten werden, da der Nitrieransatz hohe Konzentrationen sowohl an Nitrier- als auch an Nitrosierungsmitteln enthält.

Erfindungsgegenstand ist somit ein Verfahren zur Entfernung von Nitrosierungsmittel(n) aus nitrierten aromatischen Verbindungen mit einem Erstarrungspunkt zwischen etwa 10 und 120°C, das dadurch gekennzeichnet ist, dass man im Anschluss an die Nitrierung den auf einer Temperatur oberhalb der Erstarrungstemperatur der jeweiligen nitrierten aromatischen Verbindung befindlichen oder auf eine solche Temperatur gebrachten Nitrieransatz auf eine Temperatur unterhalb dieser Erstarrungstemperatur abkühlt oder abkühlen lässt und die dabei auskristallisierende nitrierte aromatische Verbindung auf übliche Weise abtrennt und neutral wäscht.

Die erfindungsgemässe Kristallisation kann unmittelbar nach der (in einer oder in mehreren Stufen durchgeführten) Nitrierung oder auch später erfolgen. Wichtig ist nur, dass die nitrierte aromatische Verbindung aus dem diese Verbindung in flüssiger bzw. geschmolzener Form enthaltenden Nitrieransatz durch Abkühlen auskristallisieren kann. Dies ist natürlich — jedenfalls bei Normal- oder nicht wesentlich erhöhtem Druck — jedoch nur dann möglich, wenn die Erstarrungstemperatur der nitrierten aromatischen Verbindung nicht wesentlich über etwa 100 bis 120°C und nicht unter der Erstarrungstemperatur der konzentrierten Salpeter- oder Nitriersäure liegt. Als vernünftige untere Grenze für die Erstarrungstemperatur der nitrierten aromatischen Verbindungen, bei denen das Verfahren noch einigermassen funktioniert, kann etwa 10°C angegeben werden. Vorzugsweise soll der Erstarrungspunkt der jeweiligen nitrierten aromatischen Verbindung aber oberhalb von etwa 30°C, insbesondere oberhalb von etwa 50°C liegen.

Das Verfahren ist somit auf die Entfernung von Nitrosierungsmittel(n) aus praktisch allen nitrierten aromatischen Verbindungen mit einer Erstarrungstemperatur zwischen etwa 10 und 120°C anwendbar. Bevorzugte, dem Verfahren zugängliche nitrierte aromatische Verbindungen sind die unter die folgende allgemeine Formel I fallenden Verbindungen:

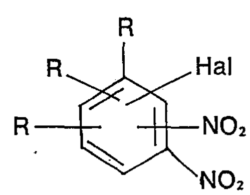

(I),

worin die Reste R unabhängig voneinander = H, ($C_1$-$C_4$)-Alkyl, $CF_3$, $SO_2NH_2$, $SO_2CH_3$, und Hal = F, Cl, Br, J, vorzugsweise Cl, Br bedeuten.

Konkrete unter diese Formel fallende Verbindungen sind z.B.

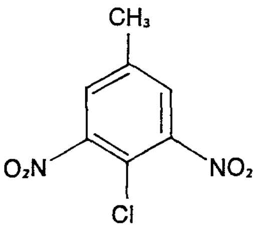,
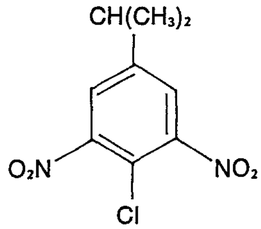,
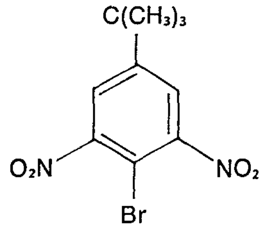,
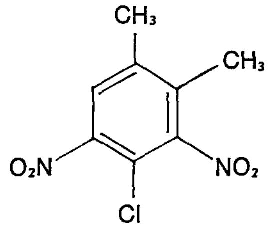,

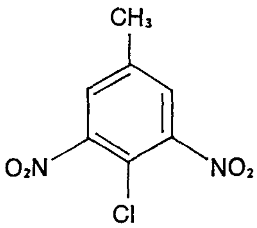,
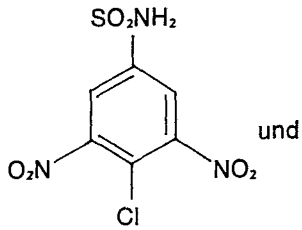
und
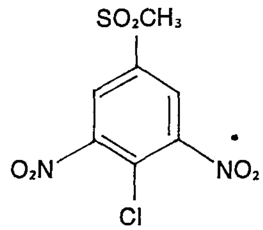.

Die für das Verfahren besonders bevorzugte nitrierte aromatische Verbindung ist das Trifluralin-Vorprodukt 4-Chlor-3,5-dinitrobenzotrifluorid.

Für die erfindungsgemässe Kristallisation beim Abkühlen des Nitrieransatzes ist es zweckmässig und vorteilhaft, wenn dabei mässig gerührt wird. Die ausgefallenen Kristalle werden dann wie üblich — z.B. durch Filtration — abgetrennt und von der anhaftenden Salpeter- oder Nitriersäure durch Neutralwaschen — z.B. mit wässriger Natronlauge und mit Wasser — befreit.

Die Nitrosierungsmittel scheinen bei der erfindungsgemässen Kristallisation praktisch vollständig in der wässrigen Phase zu verbleiben.

Wenn die so aus dem Nitrieransatz kristallisierten nitrierten aromatischen Verbindungen etwa mit Aminen weiter umgesetzt werden, lassen sich in den Umsetzungsprodukten praktisch keine Nitrosamine mehr nachweisen. Bei der Umsetzung z.B. des erfindungsgemäss aus dem Nitrieransatz kristallisierten 4-Chlor-3,5-dinitrobenzotrifluorids mit Di-n-propylamin wird ein Trifluralin erhalten, dessen Nitrosaminwerte kaum über 1 ppm liegen.

In Anbetracht des teilweise recht erheblichen Aufwands (Verbrauch zusätzlicher Chemikalien, zusätzliche Verfahrensstufen, usw.), welcher nach den Verfahren des Standes der Technik nötig ist, um z.B. zu einem weitgehend nitrosaminfreien Trifluralin zu gelangen, stellt die Einfachheit der Erfindung (lediglich Kristallisation ohne vorherige Phasentrennung, ohne Einsatz weiterer Chemikalien, keine zusätzlichen Verfahrensstufen!) einen beträchtlichen Fortschritt dar.

Das folgende Beispiel soll nun der näheren Erläuterung der Erfindung dienen. Die Nitrosaminkonzentration wurde nach den z.B. in der DE-OS 28 31 119 angegebenen Methoden bestimmt.

Beispiel

a) Herstellung von Trifluralin auf übliche Weise:

2 Mol p-Chlorbenzotrifluorid wurden in bekannter Weise zunächst mononitriert, alsdann bei 110 bis 125°C dinitriert. Nach Verdünnen der Endsäure auf eine ca. 70 gewichtsprozentige Schwefelsäure wurde bei 80°C das Nitroprodukt flüssig abgetrennt und mehrmals mit Wasser und etwas Natronlauge neutral gewaschen.

Ausbeute an 4-Chlor-3,5-dinitrobenzotrifluorid: 432 g, EP.: 54,8°C.

Nach üblicher Umsetzung mit Di-n-propylamin zum Trifluralin (Ausbeute: praktisch quantitativ, EP. 46,2°C) wurde in diesem ein Nitroso-di-n--propylamingehalt von 29 ppm ermittelt.

b) Erfindungsgemässe Kristallisation:

In gleicher Weise wie unter a) beschrieben, wurde p-Chlorbenzotrifluorid zunächst mono- und dann dinitriert. Der gesamte Nitrieransatz wurde sodann unter Rühren auf etwa 25°C abgekühlt, wobei sich das 4-Chlor-3,5-dinitrobenzotrifluorid kristallförmig abschied. Die Kristalle wurden abfiltriert und wie unter a) neutral gewaschen.

Ausbeute an 4-Chlor-3,5-dinitrobenzotrifluorid: 440 g, EP.: 55,4°C.

Nach üblicher Umsetzung mit Di-n-propylamin zum Trifluralin (Ausbeute: praktisch quantitativ, EP. 46,5°C) wurde in diesem ein Nitroso-di-n--propylamingehalt von 1 ppm ermittelt.

## Patentansprüche

1. Verfahren zur Entfernung von Nitrosierungsmittel(n) aus nitrierten aromatischen Verbindungen mit einer Erstarrungstemperatur zwischen etwa 10 und 120°C, dadurch gekennzeichnet, dass man im Anschluss an die Nitrierung den auf einer Temperatur oberhalb der Erstarrungstemperatur der jeweiligen nitrierten aromatischen Verbindung befindlichen oder auf eine solche Temperatur gebrachten Nitrieransatz auf eine Temperatur unterhalb dieser Erstarrungstemperatur abkühlt und die dabei auskristallisierende nitrierte aromatische Verbindung auf übliche Weise abtrennt und neutral wäscht.

2. Verfahren nach Anspruch 1, dadurch gekennzeichnet, dass man das oder die Nitrosierungsmittel aus nitrierten aromatischen Verbindungen mit einem Erstarrungspunkt oberhalb etwa 30°C, insbesondere oberhalb etwa 50°C, entfernt.

3. Verfahren nach den Ansprüchen 1 bis 2, dadurch gekennzeichnet, dass man das oder die Nitrosierungsmittel aus nitrierten aromatischen Verbindungen der allgemeinen Formel I

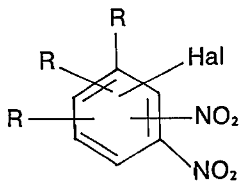

(I),

worin die Reste R unabhängig voneinander = H, ($C_1$-$C_4$)-Alkyl, $CF_3$, $SO_2NH_2$, $SO_2CH_3$, und Hal = F, Cl, Br, J, vorzugsweise Cl, Br bedeuten, entfernt.

4. Verfahren nach den Ansprüchen 1 bis 3, dadurch gekennzeichnet, dass man das oder die Nitrierungsmittel aus 4-Chlor-3,5-dinitrobenzotrifluorid entfernt.

5. Verfahren nach den Ansprüchen 1 bis 4, dadurch gekennzeichnet, dass man die Abkühlung des Nitrieransatzes unter Rühren durchführt.

**Claims**

1. Process for the removal of nitrosation agent(s) from nitrated aromatic compounds with a solidification temperature between about 10 and 120°C, characterised in cooling or allowing to cool the nitration mixture — having a temperature above the solidification temperature of the respective nitrated aromatic compound or having been heated to such a temperature — subsequent to the nitration to a temperature below this solidification temperature, separating the crystallized nitrated aromatic compound and neutral washing same in usual manner.

2. Process according to Claim 1, characterised in removing the nitrosation agent(s) from nitrated aromatic compounds with a solidification temperature higher than about 30°C, especially above about 50°C.

3. Process according to Claims 1 to 2, characterised in removing the nitrosation agent(s) from nitrated aromatic compounds of the general formula I

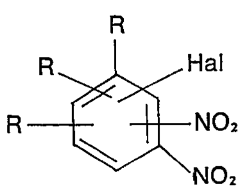

(I),

wherein the radicals R, independent from one another, mean H, ($C_1$-$C_4$)-alkyl, $CF_3$, $SO_2NH_2$, $SO_2CH_3$, and Hal is F, Cl, Br, I and preferably Cl, Br.

4. Process according to Claims 1 to 3, characterised in removing the nitrosation agent(s) from 4-chloro-3,5-dinitrobenzotrifluoride.

5. Process according to Claims 1 to 4, characterised in cooling the nitrosation mixture whilst stirring.

**Revendications**

1. Procédé pour éliminer un ou des agents de nitrosation contenus dans des composés aromatiques nitrés dont la température de solidification est comprise entre environ 10 et 120°C, procédé caractérisé en ce que, la nitration ayant été effectuée, on refroidit ou on laisse refroidir à une tmpérature inférieure à la température de solidification du composé aromatique nitré le mélange de nitration qui se trouve ou qui a été porté à une température supérieure à cette température de solidification, puis on sépare et lave à neutralité, de la manière habituelle, le composé aromatique nitré qui a alors cristallisé.

2. Procédé selon la revendication 1 caractérisé en ce qu'on élimine l'agent ou les agents de nitrosation de composés aromatiques nitrés ayant un point de solidification supérieur à environ 30°C, en particulier supérieur à environ 50°C.

3. Procédé selon l'une des revendications 1 et 2, caractérisé en ce qu'on élimine le ou les agents de nitrosation de composés aromatiques nitrés répondant à la formule générale I

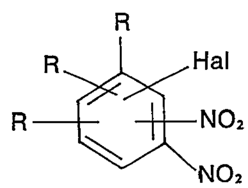

(I),

dans laquelle les symboles R représentent chacun, indépendamment les uns des autres, l'hydrogène, un alkyle en $C_1$-$C_4$, $CF_3$, $SO_2NH_2$ ou $SO_2CH_3$, et Hal représente F, Cl, Br ou I, de préférence Cl ou Br.

4. Procédé selon l'une quelconque des revendications 1 à 3, caractérisé en ce qu'on élimine le ou les agents de nitrosation du trifluorométhyl-1 dinitro-3,5 chloro-4 benzène.

5. Procédé selon l'une quelconque des revendications 1 à 4, caractérisé en ce qu'on effectue le refroidissement du mélange de nitration tout en agitant.